(19) Europäisches Patentamt
European Patent Office
Office européen des brevets

(11) **EP 3 141 896 B1**

(12) **EUROPÄISCHE PATENTSCHRIFT**

(45) Veröffentlichungstag und Bekanntmachung des Hinweises auf die Patenterteilung:
**18.10.2023  Patentblatt 2023/42**

(21) Anmeldenummer: **16188076.0**

(22) Anmeldetag: **09.09.2016**

(51) Internationale Patentklassifikation (IPC):
*C02F 1/42* (2023.01)   *C02F 1/28* (2023.01)
*C02F 1/00* (2023.01)   *G01N 33/18* (2006.01)
*B01J 49/85* (2017.01)

(52) Gemeinsame Patentklassifikation (CPC):
**C02F 1/008; B01J 49/85; C02F 1/42;**
**G01N 33/1853;** C02F 1/283; C02F 2001/425;
C02F 2001/427; C02F 2201/006; C02F 2209/006;
C02F 2209/02; C02F 2209/05; C02F 2209/055;
C02F 2209/40; C02F 2301/043; C02F 2307/10;

(Forts.)

(54) **VERFAHREN UND SYSTEM ZUM BETREIBEN EINER VORRICHTUNG ZUR BEHANDLUNG EINER WÄSSRIGEN FLÜSSIGKEIT**

METHOD AND SYSTEM FOR OPERATING A DEVICE FOR TREATING AN AQUEOUS FLUID

PROCEDE ET SYSTEME DE FONCTIONNEMENT D'UN DISPOSITIF DE TRAITEMENT D'UN LIQUIDE AQUEUX

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **10.09.2015   DE 102015115268**

(43) Veröffentlichungstag der Anmeldung:
**15.03.2017   Patentblatt 2017/11**

(60) Teilanmeldung:
**23152157.6**

(73) Patentinhaber: **BRITA SE**
**65232 Taunusstein (DE)**

(72) Erfinder: **Weidner, Peter**
**92444 Rötz (DE)**

(74) Vertreter: **van Lookeren Campagne, Constantijn August et al**
**Meissner Bolte Patentanwälte**
**Rechtsanwälte Partnerschaft mbB**
**Kaiserswerther Str. 183**
**40474 Düsseldorf (DE)**

(56) Entgegenhaltungen:
**EP-A1- 2 481 713     DE-A1-102010 003 636**
**US-A- 4 275 448**

(52) Gemeinsame Patentklassifikation (CPC): (Forts.)
C02F 2307/12

**Beschreibung**

Die Erfindung bezieht sich auf ein Verfahren zum Betreiben einer Vorrichtung zur Behandlung einer wässrigen Flüssigkeit, beispielsweise Trinkwasser, nach dem Oberbegriff des Anspruchs 1.

Die Erfindung bezieht sich ferner auf eine Vorrichtung umfassend: eine Vorrichtung zur Behandlung einer wässrigen Flüssigkeit, beispielsweise Wasser; ein System zur Verarbeitung von bei Betrieb der Behandlungsvorrichtung gewonnenen Messwerten, welches zur Durchführung eines Verfahrens der vorstehend definierten Art eingerichtet ist; und eine Sensoranordnung zur Bestimmung von Werten eines Parameters, der von der Konzentration wenigstens einer bestimmten Ionenspezies abhängig ist.

Die Erfindung bezieht sich ferner auf ein Datenverarbeitungsprogrammprodukt.

Die DE 10 2010 003 636 A1 beschreibt eine erste Wasserbehandlungsanlage, in der zulaufendes Wasser mit einem ersten Leitfähigkeitssensor vermessen wird, wobei das Messergebnis (die erste, momentane Leitfähigkeit $L_1$) an eine elektronische Steuereinheit weitergegeben wird. Das unbehandelte Wasser wird aufgeteilt auf einen Bypasszweig und auf einen Wasserbehandlungszweig. Im Wasserbehandlungszweig wird das unbehandelte Wasser über Wasserbehandlungselemente geleitet, die in einem Behälter angeordnet sind. Im Bypasszweig passiert das unbehandelte Wasser ein Verschneideventil, welches von der elektronischen Steuereinheit motorisch betätigbar ist. Die Einstellung des Verschneideventils bestimmt die Anteile von unbehandeltem Wasser und behandeltem Wasser (und damit das Verschnittverhältnis) nach einer Zusammenführung der beiden Zweige an einem Zusammenführungspunkt. Nach der Verschneidung wird mit einem zweiten Leitfähigkeitssensor die momentane Leitfähigkeit $L_2$ des Verschnittwassers vermessen und das Messergebnis an die elektronische Steuereinheit weitergeleitet. Die Steuereinheit bestimmt die verbleibende Restkapazität der Wasserbehandlungselemente. Dazu verfolgt die Steuereinheit welche Wassermenge bei welcher ersten, momentanen Leitfähigkeit behandelt wird. Die Steuereinheit vergleicht laufend, ob die (momentane) Restkapazität RK einen ersten Grenzwert $GW_1$ unterschreitet. Falls ja, wird über einen Signalgeber dieser Zustand signalisiert. Es ist weiter ein zweiter Grenzwert $GW_2$ hinterlegt. Falls die momentane zweite Leitfähigkeit $L_2$ des teilbehandelten Wassers um mehr als den zweiten Grenzwert $GW_2$ von einem Sollwert abweicht, wird ebenfalls durch den Signalgeber dieser Zustand signalisiert. Die DE 10 2010 003 636 A1 beschreibt auch eine zweite Wasserbehandlungsanlage, die lediglich einen einzigen Leitfähigkeitssensor aufweist, welcher ablaufseitig angeordnet ist. Im normalen Betrieb dieser Wasserbehandlungsanlage kann mit dem Leitfähigkeitssensor die zweite, momentane Leitfähigkeit $L_2$ des teilbehandelten (oder vollbehandelten) Wassers bestimmt werden. Zur Messung der ersten Leitfähigkeit $L_1$ des unbehandelten Wassers wird zeitweilig ein Ventil geschlossen und das Verschneideventil geöffnet, so dass unbehandeltes Wasser am Leitfähigkeitssensor vorbei fließt.

Es wird also signalisiert, dass die Behandlungselemente verbraucht sind, wenn eins von zwei alternativen Kriterien vorliegt. Ein erstes Kriterium ist, ob die auf Basis der bei der ersten Leitfähigkeit $L_1$ behandelten Menge an Wasser bestimmte Restkapazität zu gering ist. Ein zweites Kriterium ist, ob die zweite Leitfähigkeit $L_2$ zu viel von einem Sollwert abweicht. Das erste Kriterium hat als Nachteil, dass die tatsächliche Restkapazität bis zur Erschöpfung von einigen Unwägbarkeiten wie Bettvolumen, Füllvolumen, Flussgeschwindigkeit usw. abhängt. Es muss deshalb zur Sicherheit ein ziemlich hoher erster Grenzwert $GW_1$ genommen werden. Das zweite Kriterium berücksichtigt bei einem konstanten Sollwert nicht die Möglichkeit, dass sich die Zusammensetzung des unbehandelten Wassers geändert hat, mit Fehlalarmen als Folge. Bei einem von der ersten Leitfähigkeit $L_1$ abhängigen Sollwert muss auch ständig der Wert der ersten Leitfähigkeit $L_1$ bestimmt und verarbeitet werden.

Die EP 2 481 713 A1 offenbart ein Verfahren zum Betrieb einer Wasserenthärtungsanlage mit einer Enthärtungsvorrichtung umfassend ein Ionenaustauschermaterial, einer automatisch verstellbaren Verschneideeinrichtung zum Mischen eines Verschnittwasserstroms aus einem ersten, enthärteten Teilstrom und einem zweiten, rohwasserführenden Teilstrom, einem ersten Leitfähigkeitssensor im Rohwasserbereich, einem zweiten Leitfähigkeitssensor im Bereich des enthärteten Wassers und einer elektronischen Steuereinrichtung. Eine bevorzugte Variante sieht vor, dass die Regeneration des Ionentauschermaterials gestartet oder die Erschöpfung des Ionentauschermaterials signalisiert wird, wenn die Leitfähigkeit des enthärteten Wassers abnimmt bei konstant bleibender Leitfähigkeit des Rohwassers.

Es ist Aufgabe der vorliegenden Erfindung, ein Verfahren, eine Vorrichtung und ein Datenverarbeitungsprogrammprodukt der eingangs bezeichneten Art anzugeben, die eine verhältnismäßig effiziente Feststellung einer sich abzeichnenden Erschöpfung der Behandlungskapazität ermöglichen.

Diese Aufgabe wird gemäß einem ersten Aspekt durch das in Anspruch 1 definierte Verfahren gelöst.

Die Vorrichtung umfasst eine Einrichtung zum Ändern der Konzentration wenigstens einer bestimmten Ionenspezies, welche Einrichtung eine begrenzte Behandlungskapazität aufweist. Insbesondere kann die Einrichtung wenigstens ein Medium zur Behandlung der Flüssigkeit in einem Diffusionsprozess, wie Sorption (inkl. Ionenaustausch) oder Eluierung enthalten. Der Zustand, dessen Vorliegen festgestellt wird, braucht nicht der Zustand der Erschöpfung der Behandlungskapazität zu sein. Er kann ein bestimmter Zustand sein, der im Laufe der Lebensdauer der Behandlungseinrichtung auftritt. Ein Beispiel ist die Feststellung, dass nur noch eine bestimmte Restkapazität vorhanden ist. Ein anderes Beispiel tritt bei Behandlungseinrichtungen auf, die einen oder mehrere Ionenaustauscher umfassen. Im Laufe der Zeit lässt die Behandlungseffektivität nach, wobei irgendwann "Schlupf" auftritt. Dies heißt, dass nicht mehr alle Zielionen aus der Flüssigkeit entfernt werden.

Im Verfahren werden Werte eines Parameters, der von der Konzentration wenigstens der bestimmten Ionenspezies abhängig ist, gemessen. Der Parameter kann von der Konzentration nur dieser Ionenspezies oder von der Konzentration von mehr als nur diesen Ionenspezies, beispielsweise von allen Ionenspezies, abhängig sein. Zur Vereinfachung wird zwischen Messungen eines ersten und Messungen eines zweiten Typs unterschieden, wobei diese sich durch die Zusammensetzung der Flüssigkeit, an der sie vorgenommen werden, unterscheiden. Im Übrigen wird in beiden Fällen der Wert des Parameters, der von der Konzentration wenigstens der bestimmten Ionenspezies abhängig ist, bestimmt. Messungen eines ersten Typs werden wiederholt an Flüssigkeit vorgenommen, von der durch die Einrichtung behandelte Flüssigkeit wenigstens einen Anteil, beispielsweise eine Mehrheit, ausmacht. Sie kann auch die Gesamtheit der Flüssigkeit, an der die Messung vorgenommen wird, bilden. In der Regel wird die Messung des ersten Typs jedoch eine Mischung aus durch die Einrichtung behandelter und durch die Einrichtung unbehandelter Flüssigkeit betreffen. Die durch die Einrichtung unbehandelte Flüssigkeit kann in anderer Weise behandelt worden sein, beispielsweise ohne dass sich dabei die Ionenkonzentration geändert hat. In beiden Fällen handelt es sich um Flüssigkeit, die zunächst über einen oder mehrere Einlässe der Vorrichtung zugeführt worden ist. Die Messungen des ersten Typs werden an Flüssigkeit vorgenommen, die einem Auslass der Vorrichtung zugeführt wird. Die Messungen des zweiten Typs werden an nur durch die Einrichtung unbehandelter Flüssigkeit vorgenommen. Dadurch, dass wenigstens ein bei einer Messung des zweiten Typs erhaltener Messwert in Reaktion auf eine Feststellung, dass sich der bei den Messungen des ersten Typs erhaltene Wert außerhalb bestimmter Grenzen bewegt hat, zur Bestimmung, ob der Zustand vorliegt, verwendet wird, kann zwischen Änderungen im Ergebnis der Messungen des ersten Typs, die auf eine Zustandsänderung der Behandlungseinrichtung und Änderungen, die auf eine Änderung der Zusammensetzung der der Vorrichtung zugeführten Flüssigkeit zurückzuführen sind, unterschieden werden. Der Vergleich wird erst in Reaktion auf eine Feststellung, dass sich der bei den Messungen des ersten Typs erhaltene Wert außerhalb bestimmter Grenzen bewegt hat, vorgenommen. Dies macht das Verfahren relativ effizient. Es wird für den Vergleich das Ergebnis einer aktuellen Messung des zweiten Typs genommen. Dieser Wert wird mit einem älteren Wert oder einem Wert, der auf mehreren älteren Werten basiert, verglichen. Ist die Abweichung gering, hat sich der Zustand der Behandlungseinrichtung geändert. Erfindungsgemäß wird zum Erhalten eines der wenigstens einen bei einer Messung des zweiten Typs erhaltenen Messwerte in Reaktion auf eine Feststellung, dass sich der bei den Messungen des ersten Typs erhaltene Wert außerhalb bestimmter Grenzen bewegt hat, eine Messung des zweiten Typs durchgeführt.

Hierdurch wird ein aktuelles Ergebnis einer Messung des zweiten Typs erhalten. Es kann deshalb verlässlicher festgestellt werden, ob eine Änderung des bei den Messungen des ersten Typs erhaltenen Werts durch eine Zustandsänderung der Behandlungseinrichtung oder durch eine Änderung in der Zusammensetzung der der Vorrichtung zugeführten Flüssigkeit verursacht ist. Ferner ist diese Ausführungsform verhältnismäßig effizient. Es brauchen nicht regelmäßig Messungen des zweiten Typs durchgeführt und deren Ergebnisse zum Abgleich vorgehalten zu werden. Stattdessen wird ein aktuelles Messergebnis nur bei Bedarf erzeugt.

In einer Ausführungsform wird das Vorliegen des Zustands festgestellt, wenn ein letzter bei einer Messung des zweiten Typs erhaltener Messwert höchstens binnen bestimmten Grenzen von einem Wert abweicht, der auf wenigstens einem bei einer vorherigen Messung des zweiten Typs erhaltenen Wert basiert.

Dieses Kriterium bedeutet, dass die Flüssigkeit, die in der Einrichtung behandelt wurde, noch im Wesentlichen dieselbe Zusammensetzung hat. Es muss sich folglich die Effektivität der Behandlung durch die Einrichtung geändert haben.

In einer Ausführungsform werden die Messungen des ersten Typs und die Messungen des zweiten Typs mit einem selben Sensor vorgenommen. Zur Durchführung einer Messung des zweiten Typs, wird Flüssigkeit mit durch die Einrichtung unbehandelter Flüssigkeit dem Sensor zugeführt, statt Flüssigkeit mit den für die Messungen des ersten Typs vorgesehenen Anteilen. Die Benutzung nur eines einzigen Sensors macht das Verfahren günstiger in der Umsetzung. Außerdem hat Sensordrift, der für mehrere Sensoren unterschiedlich ausfallen kann, keine oder weniger Auswirkung auf das Verfahren.

Es werden die Messungen des zweiten Typs an Flüssigkeit vorgenommen, die nur aus durch die Einrichtung unbehandelter Flüssigkeit besteht.

Hierdurch wirkt sich eine Änderung in der Zusammensetzung der der Vorrichtung zugeführten Flüssigkeit stärker auf die bei den Messungen des zweiten Typs erhaltenen Messergebnisse aus.

In einer Ausführungsform werden zur Feststellung, ob sich der bei den Messungen des ersten Typs erhaltene Wert außerhalb bestimmter Grenzen bewegt hat, mehrere zuletzt erhaltene Messwerte berücksichtigt. Beispielsweise wird ein Mittelwert aus wenigstens zwei zuletzt erhaltenen Messwerten gebildet.

Somit kann ein einzelner Ausreißer im Ergebnis der wiederholt durchgeführten Messungen des ersten Typs nicht gleich zu einer Analyse der Messwerte aus den Messungen des zweiten Typs, gegebenenfalls erst zu einer Durchführung einer Messung des zweiten Typs überhaupt, führen. Es werden zum Beispiel N letzte Werte betrachtet, wobei N eine natürliche Zahl größer als eins ist. Erst, wenn die N letzten Werte alle innerhalb eines Bands um ihren Mittelwert liegen, wird der letzte (aktuellste) Wert oder der Mittelwert mit einem älteren Wert oder einem auf mehreren älteren Werten basierenden Vergleichswert verglichen. Ein erhöhter Partikelaustritt, welcher zu einem vorübergehenden Anstieg im durch die Ergebnisse der Messungen des ersten Typs gebildeten Messsignal führen würde, führt in dieser Variante nicht zu häufigen Analysen von Messwerten aus Messungen des zweiten Typs. Auf die durch den Partikelaustritt verursachten Schwankungen im Messsignal wird nicht reagiert.

In einer Ausführungsform ist wenigstens eine der bei der Feststellung, ob sich der bei den Messungen des ersten Typs erhaltene Wert außerhalb bestimmter Grenzen bewegt hat, benutzten Grenzen in Bezug auf einen auf wenigstens einem vorangehenden Messwert basierenden Wert definiert.

Es kann sein, dass sich die Zusammensetzung der unbehandelten Flüssigkeit mit der Zeit allmählich ändert. Eine solche Änderung würde sich auf das Ergebnis der Messungen des ersten Typs auswirken, ist aber zu unterscheiden von Zustandsänderungen der Einrichtung. Solche Zustandsänderungen führen in der Regel zu einem schneller an- oder absteigenden Verlauf des durch die Ergebnisse der Messungen des ersten Typs gebildeten Signals. Daher ist es effizienter, ein Band um dieses Signals zu bilden und zu analysieren, ob das Signal sich außerhalb dieses Bands bewegt hat. Die Grenzwerte wandern also dynamisch mit dem sich aus den Messungen des ersten Typs ergebenden Signal.

In einer Variante dieser Ausführungsform wird zur Feststellung, ob sich der bei den Messungen des ersten Typs erhaltene Wert außerhalb bestimmter Grenzen bewegt hat, ein für die Ableitung eines durch die Messungen des ersten Typs erhaltenen Signals nach der Zeit repräsentativer Wert bestimmt.

Statt Messwerte oder aus diesen gebildete, gegebenenfalls gewichtete Mittelwerte miteinander zu vergleichen, wird also die Steigung direkt analysiert und betrachtet, ob sie innerhalb eines bestimmten Bereichs liegt. Die Grenzen dieses Bereichs können fest, also konstant, sein.

Erfindungsgemäß umfasst die Vorrichtung eine Verschneideeinrichtung zum Verschneiden durch die Einrichtung behandelter Flüssigkeit mit durch die Einrichtung unbehandelter Flüssigkeit, wobei ein volumenbezogener Verschnittanteil der unbehandelten Flüssigkeit in der Mischung veränderbar ist.

Es ist somit möglich, unterschiedlichen Anforderungen an durch die Vorrichtung gelieferte Flüssigkeit gerecht zu werden, ohne dass die Effektivität der Behandlungseinrichtung selbst regelbar oder gar änderbar sein muss. Es ist ferner möglich, im Laufe der Lebensdauer der Einrichtung eine nachlassende Effektivität zu kompensieren.

In einer Ausführungsform wird der Verschnittanteil in Abhängigkeit zumindest der Ergebnisse der Messungen des ersten Typs angepasst.

Die Messungen des ersten Typs werden in diesem Fall an der Flüssigkeit vorgenommen, die auch an einem Auslass der Vorrichtung bereitgestellt wird. Die Messergebnisse werden folglich sowohl für die Regelung oder Steuerung des Verschnittanteils als auch für eine Überwachung des Zustands der Behandlungseinrichtung verwendet.

In einer besonderen Variante dieser Ausführungsform wird in Reaktion auf eine Feststellung, dass der Zustand vorliegt, ein anderes Verfahren zur Anpassung des Verschnittanteils in Abhängigkeit zumindest der Ergebnisse der Messungen des ersten Typs angewendet.

Wenn die Effektivität der Behandlung durch die Einrichtung bekannt ist, beispielsweise in etwa 100 % beträgt, kann die Zusammensetzung der durch die Vorrichtung gelieferten Flüssigkeit einfach durch Einstellen eines bestimmten Verschnittanteils eingestellt werden. Die Einrichtung nimmt zum Beispiel alle Ionen einer bestimmten Spezies aus der Flüssigkeit heraus. Wenn die Hälfte im Endprodukt verbleiben sollen, kann einfach ein Verschnittanteil von 50 % eingestellt werden. Ist aber nicht mehr sicher, dass die Einrichtung alle Ionen entfernt, wird ein anderes Verfahren benötigt. Mit dem vorliegenden Verfahren kann festgestellt werden, dass ein Zustand eingetreten ist, in der die Effektivität der Einrichtung nicht mehr einer einem anfänglichen Verfahren zugrundeliegenden Annahme entspricht. Es wird daraufhin ein anderes Verfahren zur Einstellung des Verschnittanteils eingesetzt.

Erfindungsgemäß werden die Messungen des zweiten Typs mit einem stromabwärts eines Zusammenführungspunkts zum Zusammenführen der durch die Einrichtung unbehandelten und der durch die Einrichtung behandelten Flüssigkeit angeordneten Sensor durchgeführt.

Es braucht also nicht in oder direkt stromabwärts der Behandlungseinrichtung ein Sensor angeordnet zu werden. Außerdem kann die Zusammensetzung der Mischung aus durch die Einrichtung behandelter und durch die Einrichtung unbehandelter Flüssigkeit an eine bestimmte Anwendung in einem durch die Vorrichtung belieferten Gerät angepasst werden. Ferner kann durch Veränderung des Verschnittanteils und Messung der dadurch verursachten Veränderung des Parameterwertes die Konzentration durch die Behandlungseinrichtung aus der Flüssigkeit entfernbarer Ionenspezies bestimmt werden. Dies ist allerdings nur möglich, wenn der Zustand der Behandlungseinrichtung derart ist, dass von einer bestimmten Behandlungseffektivität ausgegangen werden kann. Mit dem hier vorgestellten Verfahren kann festgestellt werden, ob dieser Zustand tatsächlich noch vorliegt.

Erfindungsgemäß wird zur Durchführung der Messung des zweiten Typs der Verschnittanteil derart verändert, dass ein Flüssigkeitsstrom durch die Einrichtung zum Ändern der Konzentration wenigstens einer Ionenspezies unterbrochen wird.

Es ist deshalb möglich, mit nur einem Sensor sowohl die Messungen des ersten als die des zweiten Typs durchzuführen. Da nur unter bestimmten Bedingungen das Ergebnis einer aktuellen Messung des zweiten Typs benötigt wird, braucht der Verschnittanteil nicht ständig über einen großen Bereich verändert zu werden.

In einer Ausführungsform wird durch die Einrichtung zum Ändern der Konzentration wenigstens einer Ionenspezies unbehandelte Flüssigkeit durch eine weitere Behandlungseinrichtung geführt, wobei die in der Einrichtung und der weiteren Behandlungseinrichtung durchgeführte Behandlung sich in zumindest einem der Aspekte Art und Intensität unterscheiden, beispielsweise die Behandlung in der weiteren Behandlungseinrichtung die Ionenkonzentration im Wesentlichen unverändert lässt. Hierdurch braucht der Vorrichtung zugelieferte Flüssigkeit nicht solch hohe Anforderungen zu erfüllen. Beispielsweise können in der weiteren Behandlungseinrichtung organische oder mikrobiologische Verunreinigungen zumindest teilweise entfernt werden. Da die Messung des zweiten Typs an Flüssigkeit durchgeführt wird, die zu einem größeren Anteil, nämlich in etwa vollständig, aus nicht durch die Behandlungseinrichtung behandelter Flüssigkeit besteht, kann durch kumulative Überprüfung der Änderung des Parameterwertes bei der Messung des ersten Typs und der Messung des zweiten Typs dennoch zwischen Änderungen im Zustand der Behandlungseinrichtung und Änderungen in der Zusammensetzung der der Vorrichtung zugeführten Flüssigkeit unterschieden werden. Die Behandlungen in der Behandlungseinrichtung und in der weiteren Behandlungseinrichtung brauchen sich nur in zumindest einem der Aspekte Art und Intensität in konstanter Weise zu unterscheiden. Zwischen Änderungen im Zustand der Behandlungseinrichtung und Änderungen in der Zusammensetzung der der Vorrichtung zugeführten Flüssigkeit kann freilich leichter unterschieden werden, wenn die Behandlung in der weiteren Behandlungseinrichtung die Ionenkonzentration im Wesentlichen unverändert lässt.

In einer Ausführungsform des Verfahrens ist die Einrichtung zum Ändern der Konzentration wenigstens einer bestimmten Ionenspezies in einer austauschbaren Kartusche umfasst.

Da die Behandlungskapazität endlich ist, ist die Einrichtung irgendwann zu regenerieren. Wenn sie in einer Kartusche umfasst ist, braucht die Regeneration nicht vor Ort stattzufinden.

In einer Ausführungsform des Verfahrens umfasst die Einrichtung zum Ändern der Konzentration wenigstens einer bestimmten Ionenspezies ein Medium für die Behandlung von Flüssigkeit durch Ionenaustausch.

Dies ist eine Anwendung, für die das Verfahren gut geeignet ist.

In einer Ausführungsform des Verfahrens werden die Messungen wenigstens eines des ersten und des zweiten Typs mit wenigstens einem Leitfähigkeitssensor durchgeführt.

Der Sensor kann ionenspezifisch sein, d.h. nur den Beitrag bestimmter Ionenspezies an die gesamte spezifische Leitfähigkeit messen. Er kann aber auch einfach die spezifische Leitfähigkeit messen. Das Messsignal kann zur Berücksichtigung der Tatsache, dass die spezifische Leitfähigkeit von der temperaturabhängigen Ionenaktivität abhängt, in einen Wert umgerechnet werden, der bei einer bestimmten Referenztemperatur vorherrschen würde. Das so bearbeitete Signal ist nur noch von der Konzentration abhängig.

Gemäß einem zweiten Aspekt wird eine in Anspruch 10 näher definierte Vorrichtung angegeben.

Das System ist zur Durchführung eines erfindungsgemäßen Verfahrens eingerichtet. Es signalisiert nicht bereits eine Zustandsänderung, wenn es festgestellt hat, dass sich der bei den Messungen des ersten Typs erhaltene Wert außerhalb bestimmter Grenzen bewegt hat. Es benötigt aber auch nicht eine Reihe von Messwerten aus Messungen des zweiten Typs.

Das System bewirkt in Reaktion auf eine Feststellung, dass sich der bei den Messungen des ersten Typs erhaltene Wert außerhalb bestimmter Grenzen bewegt hat, eine Messung des zweiten Typs.

Besonders am Anfang der Lebensdauer der Einrichtung braucht deshalb nicht so häufig eine Messung des zweiten Typs durchgeführt zu werden. Gemäß einem weiteren Aspekt wird mit der Erfindung ein Datenverarbeitungsprogrammprodukt angegeben, welches in einem computerlesbaren Medium aufgenommen ist, umfassend einen Befehlssatz, welcher bei Ausführung durch ein Datenverarbeitungssystem zur Verarbeitung von bei Betrieb einer erfindungsgemäßen Behandlungsvorrichtung gewonnenen Messdaten bewirkt, dass das Datenverarbeitungssystem ein erfindungsgemäßes Verfahren durchführt.

Die Erfindung wird im Folgenden unter Bezugnahme auf die beigefügten Figuren näher erläutert. Es zeigen:

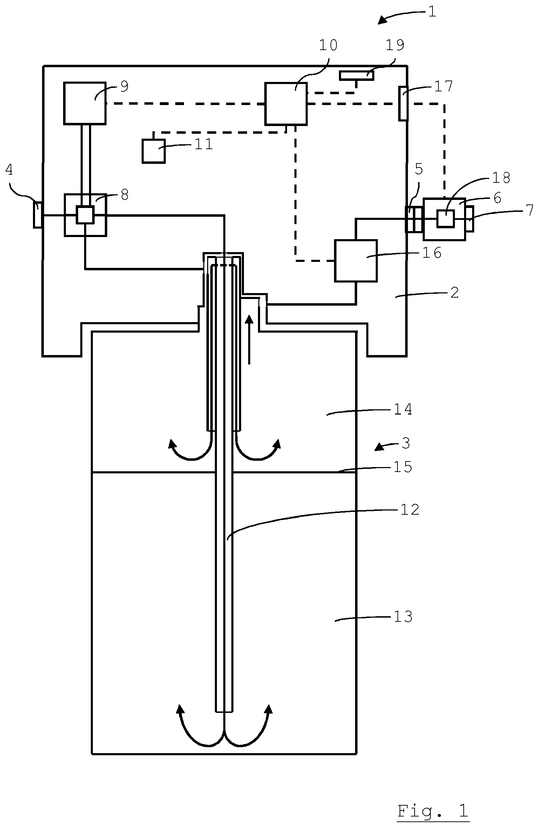

Fig. 1      eine schematische Darstellung einer Vorrichtung zur Behandlung von Wasser;
Fig. 2A    erste Schritte eines Verfahrens zur Verarbeitung von Messdaten in der Vorrichtung; und
Fig. 2B    weitere Schritte des Verfahrens.

Eine Vorrichtung 1 zur Behandlung einer wässrigen Flüssigkeit, in diesem Beispiel Trinkwasser, umfasst einen Kopfteil 2 und eine austauschbare Kartusche 3. Der Kopfteil 2 weist einen Einlassanschluss 4 und einen Auslassanschluss 5 auf. Der Einlassanschluss 4 ist an eine (nicht dargestellte) Wasserleitung anschließbar.

In der abgebildeten Ausführungsform ist an den Auslassanschluss 5 ein Messmodul 6 gekoppelt, welches wiederum einen Auslassanschluss 7 aufweist. In einer alternativen Ausführungsform sind die Komponenten des Messmoduls 6 in den Kopfteil 2 integriert. Der Modulauslassanschluss 7 ist an eine (nicht gezeigte) Leitung anschließbar, über die Wasser zu einem oder mehreren wasserverbrauchenden Geräten geführt werden kann. Die Kategorie solcher Geräte umfasst Dampfgarer, Geschirrspülmaschinen und Getränkemaschinen, insbesondere Maschinen zur Zubereitung eines Heißgetränks durch Extraktion. Beispiele von Geräten der letztgenannten Art sind Kaffee- und Espressomaschinen.

Das über den Einlassanschluss 4 in den Kopfteil 2 geleitete Wasser wird in einem Verschneideventil 8 in zwei Ströme aufgeteilt. Das Volumenstromverhältnis zwischen den zwei Strömen ist durch eine veränderbare Einstellung des Verschneideventils 8 bedingt. Ein Motor 9 oder anderer Aktuator ermöglicht eine automatische Änderung dieser Einstellung. Das Verschneideventil 8 kann beispielsweise ein Ventil der in der internationalen Patentanmeldung Nr. PCT/EP2015/066866 vom 23. Juli 2015 offenbarten Art sein. Alternativ kann das Verschneideventil 8 ein Ventil der in der WO 2009/101188 A1 offenbarten Art sein, angepasst, um eine Änderung seiner Einstellung mittels des Motors 9 zu ermöglichen. Der Motor 9 kann beispielsweise ein Servomotor oder Schrittmotor sein. Eine Datenverarbeitungseinheit 10 mit Zugriff auf einen Datenspeicher 11 steuert oder regelt mithilfe des Motors 9 die Einstellung des Verschneideventils 8.

Die Kartusche 3 ist über eine mechanische Schnittstelle derart mit dem Kopfteil 2 verbunden, dass erste und zweite Einlässe der Kartusche 3 flüssigkeitsführend mit einem ersten bzw. einem zweiten Auslass des Kopfteils 2, und ein Auslass der Kartusche 3 flüssigkeitsführend mit einem Einlass des Kopfteils 2 verbunden sind. Die drei Verbindungen sind voneinander getrennt. Einer der durch das Verschneideventil 8 erzeugte Ströme tritt über einen ersten der beiden Kartuscheneinlässe in die Kartusche 3 ein. Der zweite Strom tritt über den zweiten Einlass in die Kartusche 3 ein.

Einer der in die Kartusche eintretenden Ströme wird über ein Fallrohr 12 zum zu einem gegenüberliegenden Ende der Kartusche 3 geführt. Das Wasser gelangt so in ein erstes Bett 13 aus partikelförmigem Behandlungsmaterial. Im Beispiel umfasst das Behandlungsmaterial schwachsaures Kationenaustauscherharz, zumindest zu Anfang mehrheitlich in der Wasserstoffform. Das erste Bett 13 kann zusätzlich noch mit Alkali- und/oder Erdalkali-Ionen beladenes Ionenaustauscherharz zur Pufferung umfassen. Es kann ferner weitere Wasserbehandlungsmaterialien umfassen, beispielsweise Aktivkohle oder Schwermetallfänger. Mit dem ersten Bett 13 kann folglich eine Entkarbonisierung vorgenommen werden. Dies heißt, dass die Karbonathärte, auch temporäre Härte genannt, entfernt wird. Die Karbonathärte ist die Härte, die im Wesentlichen durch Magnesium- und Calciumkarbonat gebildet wird.

In einer alternativen Ausführungsform kann das erste Bett 13 eine Mischung aus Kationen- und Anionenaustauscherharz umfassen, um eine Entmineralisierung durchzuführen. In einer anderen alternativen Ausführungsform kann das erste Bett 13 ein Kationenaustauscherharz, beispielsweise ein starksaures Kationenaustauscherharz, in der Natriumform umfassen, um die Gesamthärte zu verringern.

Der zweite durch das Verschneideventil 8 erzeugte Wasserstrom wird nicht durch das erste Bett 13 geführt, sondern gelangt direkt in ein zweites Bett 14 aus partikelförmigem Material. In einem Beispiel behandelt das Material des zweiten Betts 14 das ihm ausgesetzte Wasser derart, dass die Ionenkonzentration im Wesentlichen unberührt gelassen wird. Das zweite Bett 14 kann beispielsweise Aktivkohle umfassen, um organische Verunreinigungen aus dem Wasser zu entfernen.

Das zweite Bett 14 ist durch ein flüssigkeitsdurchlässige Trennkomponente 15 vom ersten Bett 13 getrennt. Somit gelangt auch das durch das erste Bett 13 behandelte Wasser in das zweite Bett 14. Das zweite Bett 14 bildet folglich einen Zusammenführungspunkt. In ihm werden die zwei durch das Verschneideventil 8 erzeugten Strömen wieder zusammengeführt. Von ihm aus verlässt ein einziger Wasserstrom die Kartusche 3 durch den Kartuschenauslass und tritt wieder in den Kopfteil 2 ein. Der volumenbezogene Anteil an der Mischung des Wassers, das nur durch das zweite Bett 14 geflossen ist, wird hier als Verschnittanteil $x$ bezeichnet.

Vom Einlass des Kopfteils fließt das Wasser durch einen Flussmesser 16 hindurch zum Auslassanschluss 5. Der Flussmesser 16 liefert ein Signal an die Datenverarbeitungseinheit 10.

Über eine Schnittstelle 17 liefert auch das Messmodul 6 wenigstens ein Signal an die Datenverarbeitungseinheit 10. In einer Ausführungsform ist dieses Signal repräsentativ für die durch einen Leitfähigkeitssensor 18 gemessene spezifische elektrische Leitfähigkeit des Wassers stromabwärts des Zusammenführungspunkts, also vorliegend des zweiten Betts 14. In einer Variante umfasst das Messmodul 6 einen Temperatursensor zur Bestimmung der Umgebungstemperatur oder der Wassertemperatur. Das Messmodul 6 kann dazu eingerichtet sein, ein unter Berücksichtigung von Abweichungen dieser Temperatur von einem Referenzwert modifiziertes Leitfähigkeitssignal zu liefern. In einer anderen Ausführungsform liefert es ein Temperatursignal, das die Datenverarbeitungseinheit 10 zur Durchführung der Korrektur verwendet. In beiden Fällen wird der temperaturabhängige Einfluss der Ionenaktivität auf die spezifische Leitfähigkeit berücksichtigt, damit das Ergebnis im Wesentlichen nur von der Konzentration wenigstens der im ersten Bett 13 entfernten Härtebildner abhängt, im vorliegenden Beispiel wenigstens von der Gesamtkonzentration der in Lösung befindlichen Ionen. In einer alternativen Ausführungsform enthält das Messmodul 6 einen ionenselektiven Leitfähigkeitssensor 18, so dass das Signal von der Konzentration einer Untermenge aller im Wasser befindlichen Ionenspezies abhängt. Nachfolgend ist zur Vereinfachung nur von der Leitfähigkeit die Rede, worunter auch ein wegen Temperaturabweichungen korrigiertes Signal, das gegebenenfalls von einem ionenspezifischen Sensor stammen kann, verstanden wird.

Das im ersten Bett 13 enthaltene Behandlungsmaterial weist eine endliche Behandlungskapazität auf. Wenn diese erschöpft ist, muss die Kartusche 3 ausgetauscht werden. Ferner kann bereits vor der Erschöpfung ein nennenswerter Anteil der Karbonathärtebildner im durch das erste Bett 13 geführten Wasser zurückbleiben. Dieses Phänomen nennt man "Schlupf". Das Volumen an Wasser, das durch das erste Bett geführt werden kann, bevor Schlupf auftritt, hängt von verschiedenen Einflussfaktoren ab. Zu diesen gehören das Bettvolumen, die Totalkapazität des im ersten Bett 13 vorhandenen Ionenaustauscherharzes, die Karbonathärte des behandelten Wassers, die Flussgeschwindigkeit durch das erste Bett 13, das Füllvolumen der Kartusche 3 - dafür bestimmend, ob Kanalbildung auftritt - und ob die Wasserbehandlungsvorrichtung stetig oder mit vielen Unterbrechungen betrieben wird.

Über eine Schnittstelle 19 erhält die Datenverarbeitungseinheit einen Sollwert für die Karbonathärte des am Auslassanschluss 5 gelieferten Wassers. Die Schnittstelle 17 kann eine Mensch-Maschine-Schnittstelle sein. In einer anderen Ausführungsform umfasst die Schnittstelle 19 eine Schnittstelle für den Austausch von Daten mit einer externen Vorrichtung, beispielsweise einem Eingabe- oder Kommunikationsgerät oder dem Gerät, an das das Wasser geliefert wird.

Die Datenverarbeitungseinheit 10 steuert oder regelt den Verschnittanteil $x$, um möglichst den Sollwert der Karbonathärte zu erreichen. Dabei geht sie in verschiedenen Phasen über die Lebensdauer der Kartusche 3 unterschiedlich vor. In einer Anfangsphase kann gänzlich auf eine leitfähigkeitswertabhängige Einstellung verzichtet werden, beispielsweise, weil dann Calcium- und Magnesiumionen gegen Alkali und/oder Erdalkali-Ionen ausgetauscht werden. Zumindest nach Ablauf einer oder mehrerer Anfangsphasen startet eine Phase, in der mithilfe des vom Messmodul 6 gelieferten Signals die Karbonathärte des am Einlassanschluss 4 vorliegenden Wassers bestimmt und dann anhand eines Sollwerts der Karbonathärte am Auslassanschluss 5 ein Verschnittanteil $x$ bestimmt wird. Die dazu erforderliche Einstellung des Verschneideventils 8 wird mittels eines in der WO 2014/033147 A1 näher beschriebenen Verfahrens bestimmt. Diese nachfolgend als Hauptphase bezeichnete Phase dauert über die Mehrheit der Lebensdauer der Kartusche 3 an, bis festgestellt wird, dass Schlupf aufgetreten ist. Dann fängt eine neue Phase an, in der die Leitfähigkeit des Wassers am Auslassanschluss 5 direkt geregelt wird, also keine Karbonathärtebestimmung erfolgt.

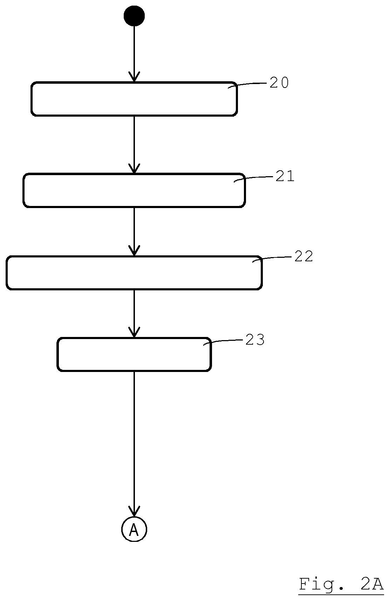

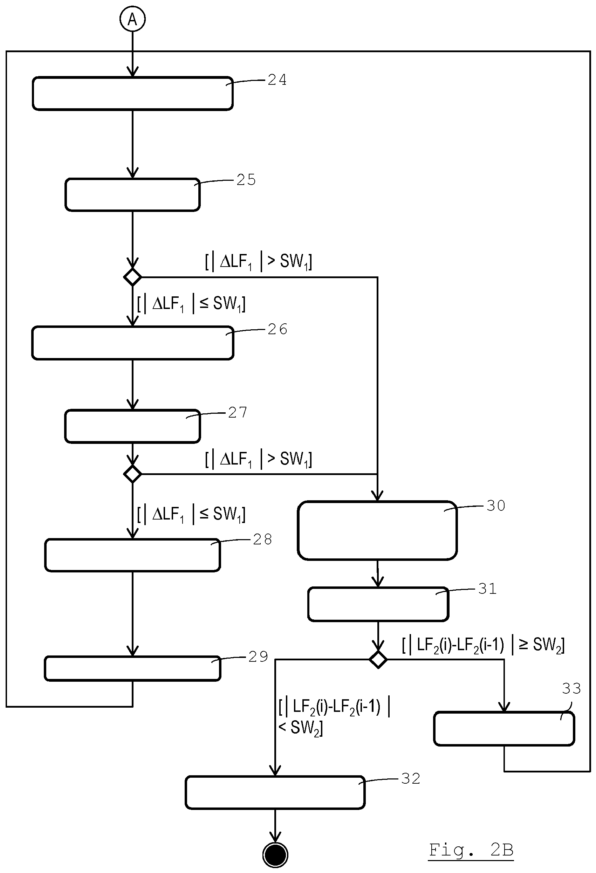

Die Hauptphase fängt (Fig. 2A) mit einem Schritt 20 an, in der der Verschnittanteil $x$ auf 100 % eingestellt wird. Dies heißt, dass der Wasserfluss durch das erste Bett 13 unterbrochen ist. Nach dem Verstellen des Verschneideventils 8 wird kurz gewartet, bis das vom Messmodul 6 gelieferte Signal einen Wert erreicht hat, der sich allenfalls in weniger als einem bestimmten Ausmaß noch ändert. Dieser Wert $LF_2(0)$ wird erfasst und gespeichert (Schritt 21). Er ist repräsentativ für die spezifische elektrische Leitfähigkeit des unbehandelten Wassers. Die Messungen bei einem Verschnittanteil $x$ von 100 % werden hier auch als Messungen des zweiten Typs bezeichnet. Sie unterscheiden sich von Messungen eines ersten Typs, die bei einem viel geringeren Verschnittanteil $x$ und im laufenden Betrieb durchgeführt werden.

Folgend auf die erste Messung des zweiten Typs wird ein erster Sollwert des Verschnittanteils $x$ bestimmt (Schritt 22). Dies kann beispielsweise ein Standardwert aus einer Tabelle oder einem Kennfeld sein, um aus der Zielkarbonathärte einen Sollwert für den Verschnittanteil $x$ herzuleiten. Der Verschnittanteil $x$ wird dann (Schritt 23) eingestellt.

Daraufhin wird wiederholt die Karbonathärte des unbehandelten Wassers bestimmt, wobei parallel überwacht wird, ob bereits Schlupf eingetreten ist.

Die Leitfähigkeit des Wassers am Auslassanschluss 5 ist von der Leitfähigkeit des unbehandelten Wassers, vom Verschnittanteil $x$ und von der Leitfähigkeit des Wassers, das im ersten Bett 13 behandelt aber noch nicht verschnitten wurde, abhängig. Wenn man den letztgenannten Leitfähigkeitswert mit $s_1$ und die des unbehandelten Wassers mit $s_0$ bezeichnet, dann beträgt die Leitfähigkeit des Wassers am Auslassanschluss 5:

$$s(x) = x \cdot s_0 + (1-x) \cdot s_1 = (s_0 - s_1) \cdot x + s_1 = \Delta s \cdot x + s_1 \qquad . \qquad (1)$$

Dabei steht $\cdot s$ für die durch die Behandlung im ersten Bett 13 bewirkte Leitfähigkeitsänderung. Dieser Wert ergibt sich aus der Ableitung der Leitfähigkeit des Wassers am Auslassanschluss 5 nach dem Verschnittanteil $x$:

$$\Delta s = s'(x). \qquad (2)$$

Die Datenverarbeitungseinheit 10 bestimmt diesen Wert durch eine Annäherung, indem der Verschnittanteil $x$ geringfügig in beiden Richtungen geändert wird und die dazugehörigen Leitfähigkeitswerte bestimmt werden:

$$\Delta s = s'(x) \approx \frac{s(x + \Delta x) - s(x - \Delta x)}{2 \cdot \Delta x}. \qquad (3)$$

Es wird also in einem ersten Schritt 24 der Verschnittanteil $x$ geringfügig erhöht. Dann wird der zugehörige Leitfähigkeitswert erfasst (Schritt 25), wobei gegebenenfalls gewartet wird, bis sich ein stabiler Wert gebildet hat. Daraufhin wird in einem nächsten Schritt 26 der Verschnittanteil $x$ verringert und wieder die Leitfähigkeit gemessen (Schritt 27). Dann wird (Schritt 28) die Leitfähigkeitsänderung -s nach der Formel (3) bestimmt.

Unter der Annahme, dass Wasser nach Behandlung im ersten Bett 13 keine Karbonathärte mehr aufweist, kann aus diesem Wert •s eine Karbonathärte *KH* des unbehandelten Wassers berechnet werden:

$$KH = F \cdot \Delta s .\qquad\qquad\qquad (4)$$

Der Umrechnungsfaktor *F* kann eine Konstante sein oder einen in einer in der WO 2014/006129 A1 näher beschriebenen Weise bestimmten, variablen Wert haben.

Sofern erforderlich wird der Verschnittanteil *x* im Anbetracht des ermittelten Werts der Karbonathärte *KH* des unbehandelten Wassers und des Zielwertes der Karbonathärte am Auslassanschluss 5 angepasst (Schritt 29). Nach Ablauf eines gewissen Zeitintervalls werden die vorstehend beschriebenen Schritte 24-29 wiederholt. Das Zeitintervall kann zum Beispiel einen oder mehrere Tage betragen. Es wird in der Regel nicht fest sein, da das Verfahren zur Bestimmung der Karbonathärte *KH* des unbehandelten Wassers davon abhängig ist, dass gerade Wasser zu einem angeschlossenen Gerät fließt.

Wie dargestellt, werden die in den Schritten 25,27 gemessenen Leitfähigkeitswerte auch dazu verwendet, um festzustellen, ob bereits Schlupf eingetreten ist. Dazu wird bei jeder Iteration einer der beiden Werte oder ein Mittelwert der beiden Werte abgespeichert und mit dem abgespeicherten Wert der vorangegangenen Iteration verglichen. Wenn die Veränderung zwischen zwei Iterationen ein bestimmtes Maximum überschreitet, wird (Schritt 30) der Verschnittanteil *x* erneut auf 100 % eingestellt, also der Wasserfluss durch das erste Bett 13 unterbrochen. Daraufhin wird (Schritt 31) die Leitfähigkeit in einer erneuten Messung des zweiten Typs gemessen. Wenn die Veränderung der Leitfähigkeit im Vergleich zum vorherigen bei einem Verschnittanteil *x* von 100 % gemessenen Wert (Schritt 21 im Falle der ersten Durchführung des Schritts 31) innerhalb bestimmter Grenzen liegt, ist Schlupf eingetreten. Es wird (Schritt 32) die Hauptphase beendet.

Wenn die Veränderung dagegen außerhalb bestimmter Grenzen liegt, hat sich die Karbonathärte *KH* des unbehandelten Wassers stark verändert. In diesem Fall wird der bei einem Verschnittanteil *x* von 100 % erhaltene Leitfähigkeitswert gespeichert (Schritt 33), und es wird die Hauptphase fortgesetzt. Der im letzten Schritt 33 abgespeicherte Wert wird das nächste Mal statt des anfangs (Schritt 21) erhaltenen Werts als Vergleichswert herangezogen.

Es kann also zwischen durch Schlupf verursachte plötzliche Leitfähigkeitswertsänderungen und solche, die auf Änderungen der Qualität des am Einlassanschluss 4 erhaltenen Wassers zurückzuführen sind, unterschieden werden. Dabei wird nur ein einziger im Messmodul 6 enthaltener Leitfähigkeitssensor 18 benutzt.

Die Erfindung ist nicht auf die vorstehend beschriebenen Ausführungsformen beschränkt, sondern kann im Rahmen der beigefügten Ansprüche variiert werden.

So ist es möglich, häufiger die aktuelle Leitfähigkeit mit einem auf wenigstens einem vorangehenden Wert basierenden Wert zu vergleichen, als nur einmal pro Iteration der Schritte 24-29 zur Bestimmung der Karbonathärte *KH* des unbehandelten Wassers. Der zum Vergleich herangezogene Wert kann auf mehreren vorangehenden Werten basieren. Beispielsweise kann er ein Mittelwert der Ergebnisse mehrerer letzter Messungen sein. Der Mittelwert kann ein gewichteter Mittelwert sein.

Auch kann in einer Ausführungsform laufend ein Mittelwert der letzten N Leitfähigkeitswerte gebildet werden, wobei N eine natürliche Zahl größer als 1 ist. Die letzten N Leitfähigkeitswerte werden auch vorübergehend abgespeichert. Nur wenn sie sich in einem relativ schmalen Band um den Mittelwert bewegen, wird der Mittelwert oder der aktuellste der N Werte mit dem zum Vergleich herangezogenen noch älteren Wert verglichen, um zu entscheiden, ob eine Messung des zweiten Typs durchgeführt werden soll.

In der dargestellten Ausführungsform wird die Leitfähigkeit mit einem auf wenigstens einem vorangehenden Wert basierenden Wert verglichen, indem die Differenz gebildet wird. Es ist auch möglich, die Ableitung nach der Zeit zu bestimmen (beispielsweise durch Bildung eines Differenzquotienten) und ihren Absolutwert als Kriterium für die Durchführung der Messung (Schritt 31) bei einem Verschnittanteil *x* von 100 % zu benutzen.

**Bezugszeichenliste**

1      - Wasserbehandlungsvorrichtung

2      - Kopfteil

3      - Kartusche

4  - Einlassanschluss

5  - Auslassanschluss

6  - Messmodul

7  - Modulauslassanschluss

8  - Verschneideventil

9  - Motor

10  - Datenverarbeitungseinheit

11  - Datenspeicher

12  - Fallrohr

13  - 1. Bett

14  - 2. Bett

15  - Trennkomponente

16  - Flussmesser

17  - Schnittstelle

18  - Leitfähigkeitssensor

19  - Schnittstelle zur Eingabe der Zielkarbonathärte

20  - Schritt (Verschnitt auf 100 % einstellen)

21  - Schritt ($LF_2(0)$ messen und abspeichern)

22  - Schritt (Sollwert $x$ des Verschnitts bestimmen)

23  - Schritt (Verschnitt $x$ einstellen)

24  - Schritt (Verschnitt auf $x+ \cdot x$ einstellen)

25  - Schritt ($LF_1(x+ \cdot x)$ messen)

26  - Schritt (Verschnitt auf $x- \cdot x$ einstellen)

27  - Schritt ($LF_1(x- \cdot x)$ messen)

28  - Schritt (Karbonathärte bestimmen)

29  - Schritt (Verschnitt ggf. anpassen)

30  - Schritt (Verschnitt auf 100 % einstellen)

31  - Schritt ($LF_2(i)$ messen)

32  - Schritt (nächste Phase einleiten)

33   - Schritt (LF$_2$(i) abspeichern)

**Patentansprüche**

**1.** Verfahren zum Betreiben einer Vorrichtung (1) zur Behandlung einer wässrigen Flüssigkeit, beispielsweise Trinkwasser,

wobei die Vorrichtung (1) eine Einrichtung (13) zum Ändern der Konzentration wenigstens einer bestimmten Ionenspezies umfasst, welche Einrichtung (13) eine begrenzte Behandlungskapazität aufweist, und

wobei die Vorrichtung (1) eine Verschneideeinrichtung (8,14) zum Verschneiden durch die Einrichtung (13) behandelter Flüssigkeit mit durch die Einrichtung unbehandelter Flüssigkeit umfasst, wobei ein volumenbezogener Verschnittanteil der unbehandelten Flüssigkeit in der Mischung veränderbar ist,

wobei Werte eines Parameters, der von der Konzentration wenigstens der bestimmten Ionenspezies abhängig ist, gemessen werden,

wobei Messungen eines ersten Typs wiederholt an Flüssigkeit vorgenommen werden, die einem Auslass (5) der Vorrichtung zugeführt wird und von der durch die Einrichtung (13) behandelte Flüssigkeit wenigstens einen Anteil ausmacht, und

wobei Messungen eines zweiten Typs an nur aus durch die Einrichtung unbehandelter Flüssigkeit bestehender Flüssigkeit vorgenommen werden, und

wobei wenigstens ein bei einer Messung des zweiten Typs erhaltener Messwert in Reaktion auf eine Feststellung, dass sich der bei den Messungen des ersten Typs erhaltene Wert außerhalb bestimmter Grenzen bewegt hat, zur Bestimmung, ob ein mit der Behandlungskapazität zusammenhängender Zustand der Einrichtung (13) vorliegt, verwendet wird, **dadurch gekennzeichnet, dass**

die Messungen des zweiten Typs mit einem stromabwärts eines Zusammenführungspunkts (14) zum Zusammenführen der durch die Einrichtung (13) unbehandelten und der durch die Einrichtung behandelten Flüssigkeit angeordneten Sensor (18) durchgeführt werden,

wobei zum Erhalten eines der wenigstens einen bei einer Messung des zweiten Typs erhaltenen Messwerte in Reaktion auf eine Feststellung, dass sich der bei den Messungen des ersten Typs erhaltene Wert außerhalb bestimmter Grenzen bewegt hat, eine Messung des zweiten Typs durchgeführt und zur Durchführung der Messung des zweiten Typs der Verschnittanteil verändert wird.

**2.** Verfahren nach Anspruch 1,
wobei das Vorliegen des Zustands festgestellt wird, wenn ein letzter bei einer Messung des zweiten Typs erhaltener Messwert höchstens binnen bestimmten Grenzen von einem Wert abweicht, der auf wenigstens einem bei einer vorherigen Messung des zweiten Typs erhaltenen Wert basiert.

**3.** Verfahren nach einem der vorhergehenden Ansprüche,
wobei die Messungen des ersten Typs und die Messungen des zweiten Typs mit einem selben Sensor (18) vorgenommen werden.

**4.** Verfahren nach einem der vorhergehenden Ansprüche,
wobei zur Feststellung, ob sich der bei den Messungen des ersten Typs erhaltene Wert außerhalb bestimmter Grenzen bewegt hat, mehrere zuletzt erhaltene Messwerte berücksichtigt werden, beispielsweise ein Mittelwert aus wenigstens zwei zuletzt erhaltenen Messwerten gebildet wird.

**5.** Verfahren nach einem der vorhergehenden Ansprüche,
wobei wenigstens eine der bei der Feststellung, ob sich der bei den Messungen des ersten Typs erhaltene Wert außerhalb bestimmter Grenzen bewegt hat, benutzten Grenzen in Bezug auf einen auf wenigstens einem vorangehenden Messwert basierenden Wert definiert ist.

**6.** Verfahren nach einem der vorhergehenden Ansprüche,
wobei zur Feststellung, ob sich der bei den Messungen des ersten Typs erhaltene Wert außerhalb bestimmter Grenzen bewegt hat, ein für die Ableitung eines durch die Messungen des ersten Typs erhaltenen Signals nach der Zeit repräsentativer Wert bestimmt wird.

**7.** Verfahren nach einem der vorhergehenden Ansprüche,
wobei der Verschnittanteil in Abhängigkeit zumindest der Ergebnisse der Messungen des ersten Typs angepasst wird.

**8.** Verfahren nach einem der vorhergehenden Ansprüche,
wobei die Einrichtung (13) zum Ändern der Konzentration wenigstens einer bestimmten Ionenspezies in einer austauschbaren Kartusche (3) umfasst ist.

**9.** Verfahren nach einem der vorhergehenden Ansprüche,
wobei die Einrichtung (13) zum Ändern der Konzentration wenigstens einer bestimmten Ionenspezies ein Medium für die Behandlung von Flüssigkeit durch Ionenaustausch umfasst.

**10.** Vorrichtung umfassend: eine Vorrichtung (1) zur Behandlung einer wässrigen Flüssigkeit, beispielsweise Wasser; ein System zur Verarbeitung von bei Betrieb der Behandlungsvorrichtung (1) gewonnenen Messwerten, beispielsweise zur Durchführung eines Verfahrens nach einem der vorhergehenden Ansprüche eingerichtet; und eine Sensoranordnung (6,18) zur Bestimmung von Werten eines Parameters, der von der Konzentration wenigstens einer bestimmten Ionenspezies abhängig ist,

wobei die Vorrichtung (1) zur Behandlung der wässrigen Flüssigkeit eine Einrichtung (13) zum Ändern der Konzentration der wenigstens einen bestimmten Ionenspezies umfasst, welche Einrichtung (13) eine begrenzte Behandlungskapazität aufweist, und
wobei die Vorrichtung (1) zur Behandlung der wässrigen Flüssigkeit eine Verschneideeinrichtung (8,14) zum Verschneiden durch die Einrichtung (13) behandelter Flüssigkeit mit durch die Einrichtung (13) unbehandelter Flüssigkeit umfasst, wobei ein volumenbezogener Verschnittanteil der unbehandelten Flüssigkeit in der Mischung veränderbar ist,
wobei das System eine Schnittstelle (17) zu der Sensoranordnung (6,18) umfasst,
wobei die Sensoranordnung (6,18) zur wiederholten Durchführung von Messungen eines ersten Typs an Flüssigkeit, von der durch die Einrichtung (13) behandelte Flüssigkeit wenigstens einen Anteil ausmacht, und zur Durchführung von Messungen eines zweiten Typs an Flüssigkeit, von der durch die Einrichtung (13) unbehandelte Flüssigkeit zumindest einen Anteil ausmacht, eingerichtet ist,
wobei das System dazu konfiguriert ist, in Reaktion auf eine Feststellung, dass sich der bei den Messungen des ersten Typs erhaltene Wert außerhalb bestimmter Grenzen bewegt hat, wenigstens einen bei einer Messung des zweiten Typs erhaltenen Messwert zur Bestimmung, ob ein mit der Behandlungskapazität zusammenhängender Zustand der Einrichtung (13) vorliegt, zu verwenden,
wobei die Sensoranordnung (6,18) einen stromabwärts eines Zusammenführungspunkts (14) zum Zusammenführen der durch die Einrichtung (13) unbehandelten und der durch die Einrichtung (13) behandelten Flüssigkeit angeordneten Sensor (18) umfasst, und
wobei das System dazu eingerichtet ist, in Reaktion auf eine Feststellung, dass sich der bei den Messungen des ersten Typs erhaltene Wert außerhalb bestimmter Grenzen bewegt hat, eine Messung des zweiten Typs mit dem stromabwärts des Zusammenführungspunkts (14) zum Zusammenführen der durch die Einrichtung (13) unbehandelten und der durch die Einrichtung (13) behandelten Flüssigkeit angeordneten Sensor (18) zu bewirken und zur Durchführung der Messung des zweiten Typs den Verschnittanteil zu verändern.

**11.** Datenverarbeitungsprogrammprodukt, welches in einem computerlesbaren Medium aufgenommen ist, umfassend einen Befehlssatz, welcher bei Ausführung durch ein Datenverarbeitungssystem zur Verarbeitung von bei Betrieb einer Behandlungsvorrichtung (1) gemäß Anspruch 10 gewonnenen Messdaten bewirkt, dass das Datenverarbeitungssystem ein Verfahren nach einem der Ansprüche 1-9 durchführt.

**Claims**

**1.** Method for operating a device (1) for treating an aqueous fluid, for example drinking water,

wherein the device (1) comprises an apparatus (13) for changing the concentration of at least one specified ion species, which apparatus (13) has a limited treatment capacity, and
wherein the device (1) comprises a blending apparatus (8, 14) for blending fluid treated by the apparatus (13) with fluid which has not been treated by the apparatus, wherein a blending proportion related to volume of the non-treated fluid in a mixture can be changed,
wherein values are measured of a parameter which is a function of the concentration of at least the specified ion species,

wherein measurements of a first type are repeatedly undertaken on fluid which is supplied to an outlet (5) of the device and constitutes at least a part of the fluid treated by the apparatus (13), and

wherein measurements of a second type are undertaken on only fluid consisting of fluid which is not treated by the apparatus, and

wherein at least one measurement value obtained from a measurement of the second type in reaction to a determination that the value obtained in the measurements of the first type was outside specified limits, is used in order to specify whether a state of the apparatus (13) is present which is related to the treatment capacity, **characterised in that**

the measurements of the second type are carried out with a sensor (18) arranged downstream of a merging point (14) for merging the fluid which is not treated by the apparatus (13) and the fluid treated by the apparatus, wherein for obtaining one of the at least one measurement values obtained in a measurement of the second type in reaction to a determination that the value obtained in the measurements of the first type was outside specified limits, a measurement of the second type is carried out and, for carrying out the measurement of the second type, the blending proportion is changed.

2. Method according to claim 1,
wherein the presence of the state is determined when a last measurement value obtained at a measurement of the second type deviates at most within specified limits from a value which is based on at least one value obtained in a previous measurement of the second type.

3. Method according to any of the preceding claims,
wherein the measurements of the first type and the measurements of the second type are undertaken with the same sensor (18).

4. Method according to any of the preceding claims,
wherein in order to determine whether the value obtained during the measurements of the first type was outside specified limits, several most recently obtained measurement values are taken into consideration, for example an average value is formed from at least two most recently obtained measurement values.

5. Method according to any of the preceding claims,
wherein at least one of the limits used in the determination whether the value obtained during the measurements of the first type was outside specified limits is defined with relation to a value based on at least one previous measurement value.

6. Method according to any of the preceding claims,
wherein for determining whether the value obtained in the measurements of the first type was outside specified limits, a value which is representative for the derivation of a signal obtained by means of the measurements of the first type according to the time is specified.

7. Method according to any of the preceding claims,
wherein the blending proportion is adapted as a function at least of the results of the measurements of the first type.

8. Method according to any of the preceding claims,
wherein the apparatus (13) for changing the concentration of at least one specified ion species is comprised in an exchangeable cartridge (3).

9. Method according to any of the preceding claims,
wherein the apparatus (13) for changing the concentration of at least one specified ion species comprises a medium for the treatment of fluid by means of ion exchange.

10. Device comprising: a device (1) for treating an aqueous fluid, for example water; a system configured for processing measurement values obtained by operating the treatment device (1), for example for carrying out a method according to any of the preceding claims; and a sensor arrangement (6, 18) for specifying values of a parameter which is dependent on the concentration of at least one specified ion species,

wherein the device (1) for treating the aqueous fluid comprises an apparatus (13) for changing the concentration of the at least one specified ion species, which apparatus (13) has a limited treatment capacity, and wherein the device (1) for treating the aqueous fluid comprises a blending apparatus (8, 14) for blending fluid treated by the apparatus (13) with fluid which is not treated by the apparatus (13), wherein a volume-related blending proportion of the non-treated fluid in a mixture can be changed,

wherein the system comprises an interface (17) to the sensor arrangement (6, 18),

wherein the sensor arrangement (6, 18) is configured to repeatedly carry out measurements of a first type on fluid, of which fluid treated by the apparatus (13) makes at least a proportion, and to carry out measurements of a second type on fluid of which fluid which is not treated by the apparatus (13) makes at least a proportion,

wherein the system is configured, in reaction to a determination that the value obtained in the measurements of the first type was outside specified limits, to use at least one measurement value obtained in a measurement of the second type for specifying whether a state of the apparatus (13) is present which correlates with the treatment capacity,

wherein the sensor arrangement (6, 18) comprises a sensor (18) arranged downstream of a merging point (14) for merging the fluid which is not treated by the apparatus (13) and the fluid treated by the apparatus (13), and wherein the system is configured, in reaction to a determination that the value obtained in the measurements of the first type was outside specified limits, to effect a measurement of the second type with the sensor (18) arranged downstream of the merging point (14) for merging the fluid which is not treated by the apparatus (13) and the fluid treated by the apparatus (13) and for carrying out a measurement of the second type to change the blending proportion.

11. Data processing program product which is accommodated in a computer readable medium, comprising a set of commands which when executed by a data processing system for processing measurement data obtained during the operation of a treatment device (1) according to claim 10 causes the data processing system to carry out a method according to any of claims 1-9.

## Revendications

1. Procédé de fonctionnement d'un dispositif (1) pour le traitement d'un liquide aqueux, par exemple de l'eau potable,

   dans lequel le dispositif (1) comprend un appareil (13) pour modifier la concentration d'au moins une espèce d'ion déterminée, lequel appareil (13) présente une capacité de traitement limitée, et

   dans lequel le dispositif (1) comprend un appareil de mélange (8, 14) pour mélanger un liquide traité par l'appareil (13) au liquide non traité par l'appareil, dans lequel une proportion de mélange liée au volume du liquide non traité peut être modifiée dans le mélange,

   dans lequel des valeurs d'un paramètre qui dépend de la concentration d'au moins l'espèce d'ion déterminée sont mesurées,

   dans lequel des mesures d'un premier type sont effectuées de manière répétée sur un liquide qui est amené à une sortie (5) du dispositif et représente au moins une proportion du liquide traité par l'appareil (13), et

   dans lequel des mesures d'un second type sont effectuées sur uniquement le liquide se composant du liquide non traité par l'appareil, et

   dans lequel au moins une valeur de mesure obtenue lors d'une mesure du second type est utilisée en réponse à une détermination que la valeur obtenue lors des mesures du premier type s'est déplacée en dehors de limites déterminées, afin de déterminer s'il existe un état lié à la capacité de traitement de l'appareil (13), **caractérisé en ce que**

   les mesures du second type sont réalisées avec un capteur (18) disposé en aval d'un point de fusion (14) pour fusionner le liquide non traité par l'appareil (13) et le liquide traité par l'appareil,

   dans lequel pour obtenir une des au moins une valeur de mesure obtenue lors d'une mesure du second type en réponse à une détermination que la valeur obtenue lors des mesures du premier type s'est déplacée en dehors de limites déterminées, une mesure du second type est réalisée et pour réaliser la mesure du second type, la proportion de mélange est modifiée.

2. Procédé selon la revendication 1,
   dans lequel la présence de l'état est établie lorsqu'une dernière valeur de mesure obtenue lors d'une mesure du second type s'écarte tout au plus à l'intérieur de limites déterminées d'une valeur qui est basée sur au moins une valeur obtenue lors d'une mesure précédente du second type.

3. Procédé selon l'une quelconque des revendications précédentes,
   dans lequel les mesures du premier type et les mesures du second type sont effectuées avec un même capteur (18).

**4.** Procédé selon l'une quelconque des revendications précédentes,
dans lequel, pour déterminer si la valeur obtenue lors des mesures du premier type s'est déplacée en dehors de limites déterminées, plusieurs dernières valeurs de mesure obtenues sont prises en compte, par exemple une valeur moyenne est formée à partir d'au moins deux dernières valeurs de mesure obtenues.

**5.** Procédé selon l'une quelconque des revendications précédentes,
dans lequel au moins l'une des limites utilisées pour déterminer si la valeur obtenue lors des mesures du premier type s'est déplacée en dehors de limites déterminées est définie par rapport à une valeur basée sur au moins une valeur de mesure précédente.

**6.** Procédé selon l'une quelconque des revendications précédentes,
dans lequel pour déterminer si la valeur obtenue lors des mesures du premier type s'est déplacée en dehors de limites déterminées, une valeur représentative pour la dérivation d'un signal obtenu au moyen des mesures du premier type en fonction du temps est déterminée.

**7.** Procédé selon l'une quelconque des revendications précédentes,
dans lequel la proportion de mélange est adaptée en fonction au moins des résultats des mesures du premier type.

**8.** Procédé selon l'une quelconque des revendications précédentes,
dans lequel l'appareil (13) pour modifier la concentration d'au moins une espèce d'ion déterminée est compris dans une cartouche (3) remplaçable.

**9.** Procédé selon l'une quelconque des revendications précédentes,
dans lequel l'appareil (13) pour modifier la concentration d'au moins une espèce d'ion déterminée comprend un milieu pour le traitement de liquide par échange d'ions.

**10.** Dispositif comprenant : un dispositif (1) pour le traitement d'un liquide aqueux, par exemple de l'eau ; un système configuré pour le traitement de valeurs de mesure obtenues lors du fonctionnement du dispositif de traitement (1), par exemple pour la réalisation d'un procédé selon l'une quelconque des revendications précédentes ; et un agencement de capteurs (6, 18) pour la détermination de valeurs d'un paramètre qui dépend de la concentration d'au moins une espèce d'ion déterminée,

dans lequel le dispositif (1) pour le traitement du liquide aqueux comprend un appareil (13) pour modifier la concentration de l'au moins une espèce d'ion déterminée, lequel appareil (13) présente une capacité de traitement limitée, et
dans lequel le dispositif (1) pour le traitement du liquide aqueux comprend un appareil de mélange (8, 14) pour mélanger un liquide traité par l'appareil (13) au liquide non traité par l'appareil (13), dans lequel une proportion de mélange liée au volume du liquide non traité peut être modifiée dans le mélange,
dans lequel le système comprend une interface (17) vers l'agencement de capteurs (6, 18),
dans lequel l'agencement de capteurs (16, 18) est configuré pour la réalisation répétée de mesures d'un premier type de liquide qui représente au moins une proportion du liquide traité par l'appareil (13), et pour la réalisation de mesures d'un second type de liquide qui représente au moins une proportion de liquide non traité par l'appareil (13),
dans lequel le système est configuré pour utiliser, en réponse à une détermination que la valeur obtenue lors des mesures du premier type s'est déplacée en dehors de limites déterminées, au moins une valeur de mesure obtenue lors d'une mesure du second type pour déterminer s'il existe un état dépendant de la capacité de traitement de l'appareil (13),
dans lequel l'agencement de capteurs (6, 18) comprend un capteur (18) disposé en aval d'un point de fusion (14) pour fusionner le liquide non traité par l'appareil (13) et le liquide traité par l'appareil (13), et
dans lequel le système est configuré pour, en réponse à une détermination que la valeur obtenue lors des mesures du premier type s'est déplacée en dehors limites déterminées, effectuer une mesure du second type avec le capteur (18) disposé en aval du point de fusion (14) pour fusionner le liquide non traité par l'appareil (13) et le liquide traité par l'appareil (13) et pour modifier la proportion de mélange pour la réalisation de la mesure du second type.

**11.** Produit de programme de traitement des données qui est logé dans un support lisible par ordinateur, comprenant un jeu d'instructions qui, lorsqu'elles sont exécutées par un système de traitement des données pour le traitement des données de mesure obtenues lors du fonctionnement d'un dispositif de traitement (1) selon la revendication 10, amène le système de traitement des données à réaliser un procédé selon l'une quelconque des revendications 1-9.

Fig. 1

Fig. 2A

Fig. 2B

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102010003636 A1
- EP 2481713 A1
- EP 2015066866 W
- WO 2009101188 A1
- WO 2014033147 A1
- WO 2014006129 A1